# EUROPÄISCHE PATENTANMELDUNG

(11) **EP 3 954 315 A1**
(43) Veröffentlichungstag der Anmeldung: **16.02.2022**
(21) Anmeldenummer: 21195072.0
(22) Anmeldetag: 25.06.2015
(51) Int. Cl.: A61B 18/14

(54) **HF-CHIRURGISCHE RESEKTIONSSCHLINGE FÜR DIE FLEXIBLE ENDOSKOPIE**

(30) Priorität: 25.06.2014 EP 14173888
(62) Teilanmeldung aus: 15732225.6
(71) Anmelder: Farin, Günter, 72070 Tübingen (DE); endox Feinwerktechnik GmbH, 72574 Bad Urach (DE)
(72) Erfinder: Farin, Günter, 72070 Tübingen (DE)
(74) Vertreter: Lohr, Jöstingmeier & Partner

(57) **Zusammenfassung**

Eine Polypektomieschlinge mit zwei Schlingenteilen hat einen Schlingenteil, der komplett elektrisch isoliert ist und einen anderen Schlingenteil, der in wenigstens einem distalen Abschnitt elektrisch nicht isoliert ist. Weiterhin hat die Schlinge an ihrem distalen Ende mindestens eine elektrisch isolierte, HF-chirurgisch nicht aktive Gleitkufe.

## Beschreibung

### Technisches Gebiet

Die Erfindung betrifft hochfrequenzchirurgische (HF-chirurgische) Resektionsschlingen für Instrumente oder als Teil von Instrumenten zum endoskopisch kontrollierten Entfernen polypenartig oder flach wachsender pathologischer Mukosa-Areale (sog. Läsionen) des Magendarmtrakts. Diese Instrumente werden u.a. Polypektom oder - pars pro toto - Polypenschlingen genannt.

### Stand der Technik

Eines der ersten dieser Instrumente wird im Jahre 1971 im Deutschen Gebrauchsmuster Nr. 71115781 beschrieben. Heute wie bereits damals bestehen diese Instrumente im Wesentlichen aus einem flexiblen ca. 2 m langen Katheter aus einem Kunststoff, einem in dem Katheter beweglichen metallischen Manipulationsdraht, einer HF-chirurgischen metallischen Resektionsschlinge an einem - dem sog. distalen - Ende des Manipulationsdrahts und einem Manipulationsgriff am anderen - dem sog. proximalen - Ende des Manipulationsdrahts. Der Katheter mit der Resektionsschlinge dieser Instrumente werden seit mehr als 40 Jahren durch sog. Instrumentier- bzw. Arbeitskanäle flexibler Endoskope angewendet um hiermit Läsionen der Mukosa des Magendarmtrakts als Prophylaxe gegen Magen-Darm-Krebs HF-chirurgisch zu entfernen.

Wurden mit diesen Instrumenten bis vor ca. 20 Jahren nur relativ kleine Läsionen (< 2 cm) entfernt, so besteht heute der Trend, auch große Läsionen (> 2 cm) hiermit zu entfernen. Allerdings stehen diesem Trend einige Probleme entgegen. Je größer die Läsion, desto größer ist auch die Wahrscheinlichkeit eines manifesten Karzinom in dieser Läsion. Deswegen fordern Onkologen, dass insbesondere große Läsionen in sano, also bis ins angrenzende gesunde Gewebe entfernt werden. Um pathohistologisch sicher prüfen zu können, ob die Entfernung der Läsion wirklich in sano erfolgte und ob in der entfernten Läsionen bereits ein Karzinom (Krebsgewebe) vorhanden ist, und wenn ja, ob dieses Karzinom bereits in Lymph- und/oder arterielle Gefäße infiltriert ist, also bereits ein Risiko von Metastasen in anderen Organen besteht, fordern die Pathologen die Entfernung der Läsion en bloc, also in einem Stück und inklusive der unter der Läsion befindlichen Submukosa, zumindest des oberen Drittels (die sog. sm1) der Submukosa. Um diese Forderung der Pathologen zu erfüllen, ist es zweckmäßig, beispielsweise physiologische NaCI-Lösung in die Submukosa zu injizieren, so dass diese infolge Absorption der NaCl Lösung nicht nur quillt, also dicker wird, sondern auch die spezifische elektrische Leitfähigkeit der Submukosa größer wird. Da mit Rücksicht auf die o.g. Forderungen der Onkologen Läsionen mit einem zirkumferenten Sicherheitsabstand von ca. 5 mm entfernt werden müssen, muss beispielsweise bei einer 3 cm Läsion ein Mukosa-Areal mit 4 cm Durchmesser en bloc HF-chirurgisch entfernt werden.

Die HF-chirurgische en-bloc Entfernung großer Läsionen (> 2 cm) mit HF-chirurgischen Schlingen bzw. Polypektomieschlingen ist u.a. insofern problematisch, als für einen HF-chirurgischen Schnitt während der sog. Anschnittphase, also in der Zeit zwischen Aktivierung des HF-Strom-Generators und der zum HF-chirurgischen Schneiden von Geweben erforderlichen elektrischen Lichtbogen zwischen Polypektomieschlinge und Gewebe ein HF-Strom von mindestens 0,5 A_{eff} pro cm Schlingenlänge erforderlich ist, damit der Schnitt mit möglichst geringer Verzögerung, der sog. Anschnittverzögerung, entstehen kann. Bei zu langer Anschnittverzögerung besteht das Risiko, dass die Organwand unterhalb der Läsion thermisch geschädigt wird und postoperativ eine sog. post Polypektomie Perforation der Organwand entstehen kann. Da in der Endoskopie verfügbare HF-Generatoren einen HF-Strom mit maximal nur 1,5 bis 2,0 A_{eff} liefern, können hiermit nur Läsionen bei einer effektiven Schlingenlänge von 3 bis 4 cm, was einem Durchmesser von ca. 1 bis 1,5 cm entspricht, ausreichend verzögerungsfrei entfernt werden.

Ein weiteres Problem bisher verfügbarer Polypektomieschlingen besteht darin, dass sie in alle Richtungen, insbesondere auch in Richtung Organwand schneiden können. Um einen Schnitt in Richtung Organwand oder gar durch die Organwand zu vermeiden, dürfen diese Polypektomieschlingen während der Aktivierung des HF-Strom-Generators nicht gegen die Organwand gedrückt werden und die Schnittführung muss von der Organwand weg geführt werden. Dies ist insbesondere bei der HF-chirurgischen Entfernung großer sessiler Polypen sowie großer flacher Läsionen insofern problematisch, als hierdurch eine Schnittführung planparallel zur Organwand bzw. zur Muskularis propria unterhalb der Läsion nicht möglich ist, mit der Konsequenz, dass insbesondere Große Läsionen nicht komplett en-bloc entfernt werden können. Deswegen werden große Läsionen entweder mit bisher verfügbaren Polypektomieschlingen in mehreren kleineren Stücken (piece meal technique) oder mit dem in Japan entwickelten Verfahren der Endoskopischen Submukosa Dissektion (ESD) entfernt. Die piece meal technique erfüllt nicht die o.g. Forderungen der Pathologen. Die ESD ist schwierig und sehr zeitaufwendig in der Anwendung.

In der Deutschen Offenlegungsschrift DE 100 28 413 A1 werden elektrochirurgische Instrumente mit jeweils einer Elektrode, u.a. auch Schlingenelektroden, mit einem sog. Elektrodenkern und einer den Elektrodenkern partiell abdeckenden Isolierummantelung offenbart. Dabei ist an der Schlingenspitze die freie Elektrodenfläche des Elektrodenkerns symmetrisch durch eine Isolierummantelung mit einer Vielzahl von Öffnungen oder eine den Elektrodenkern nur in einem Kreissegment umschließende Isolierummantelung reduziert.

Hierdurch soll die zum Schneiden erforderliche Stromstärke reduziert werden.

Mit diesen Schlingenelektroden kann zwar das o.g. Problem einer zu langen Anschnittverzögerung beseitigt werden, die Herstellung dieser Schlingenelektroden ist jedoch durch die Segmentierung der Ummantelung sehr aufwendig.

Außerdem dürfen diese Schlingenelektroden während der Aktivierung des HF-Strom Generators und insbesondere während der HF-chirurgischen Schnittführung nicht gegen die Organwand gedrückt werden weil diese Schlingenelektroden auch in Richtung Organwand schneiden können.

Ein weiteres Problem bei Anwendung aller bisher verfügbaren Polypektomieschlingen besteht darin, das weder der Endoskopiker noch seine Assistenz (z.B. Endoskopie-Schwester) insbesondere während der HF-chirurgischen Schnittführung beobachten bzw. visuell kontrollieren kann, ob, und wenn ja, wie schnell die HF-chirurgische Resektionsschlinge schließt. Infolge der mehr oder weniger großen Elastizität des langen Katheters einerseits und Manipulationsdrahtes andererseits, sowie der beachtlichen Reibung zwischen Katheter und Manipulationsdraht, und auch infolge des bei bisher verfügbaren Polypektomen großen axialen Totweges (Hysterese) zwischen Manipulationsgriff und Resektionsschlinge, ist eine zuverlässige Kontrolle des Schließens der Schlinge am Manipulationsgriff in der Regel nicht möglich. Eine Kontrolle des Schließens der Schlinge sowie der Geschwindigkeit der Schnittführung ist aber bezüglich der Schnittqualität und insbesondere bezüglich deines schnittsynchronen Gefäßverschlusses sehr wichtig.

### Darstellung der Erfindung

Es ist Aufgabe der Erfindung, HF-chirurgische Resektionsschlingen für Instrumente oder als Teil von Instrumenten zum endoskopisch kontrollierten Entfernen polypenartig oder flach wachsender pathologischer Mukosa-Areale (sog. Läsionen) des Magendarmtrakts zu entwickeln, bei welcher die o.g. Probleme nicht vorhanden sind. Derartige Resektionsschlingen werden nachfolgend auch Schlinge genannt.

Insbesondere ist es Aufgabe der Erfindung, derartige Resektionsschlingen zu entwickeln, bei deren Anwendung die Anschnittverzögerung möglich kurz ist und die trotzdem möglichst einfach hergestellt werden können.

Es ist darüber hinaus Aufgabe der Erfindung, solche Resektionsschlingen zu entwickeln, die während der Aktivierung des HF-Generators sowie während der HF-chirurgischen Schnittführung gegen die Organwand gedrückt werden dürfen ohne in oder gar durch die Organwand zu schneiden.

Es ist darüber Hinaus Aufgabe der Erfindung, solche Resektionsschlingen zu entwickeln, bei welchen der Endoskopiker und/oder die Assistenz (z.B. Endoskopie Schwester) auch das Öffnen sowie das Schließen der Schlinge auch dann visuell kontrollieren bzw. beobachten können / kann, wenn die Schlinge um eine Läsion geschlungen nicht sichtbar ist, was insbesondere während der Schnittführung entscheidend wichtig ist.

Die Aufgabe der Erfindung wird durch eine HF-chirurgische Resektionsschlinge nach dem unabhängigen Patentanspruch 1 gelöst. Weitere Ausführungsformen sind in den abhängigen Patentansprüchen beschrieben.

Die HF-chirurgische Resektionsschlinge umfasst einen ersten Schlingenteil sowie einen zweiten Schlingenteil. Die beiden Schlingenteile umfassen einen Draht, vorzugsweise einen Volldraht, besonders bevorzugt aus Stahl oder Nitinol und sie haben jeweils ein proximales und ein distales Ende. Sie bilden mit ihren distalen Enden eine Schlingenspitze. Dabei können wahlweise zwei Schlingenteile an ihren distalen Enden zu einer Schlingenspitze miteinander verbunden sein. Alternativ kann die Schlinge auch einstückig ausgeführt sein, wobei die beiden Schlingenteile aus einem Teil bestehen. An den proximalen Enden sind die beiden Schlingenteile mit einem Manipulationsdraht verbunden, mit dem sie in einen Katheter hineingezogen und/oder aus diesem herausgeschoben werden können.

Wenigstens ein Schlingenteil ist proximal elektrisch isoliert und in einem distalen Bereich (zur Schlingenspitze hin) elektrisch nicht isoliert. Wahlweise ist ein Schlingenteil vollständig elektrisch isoliert. Eine Alternative Ausführungsform besteht darin, dass höchstens drei, besonders bevorzugt zwei nicht isolierte Bereiche vorgesehen sind. Messungen mit verschiedenen HF-Chirurgie Generatoren haben außerdem gezeigt, dass Schlingen entsprechend der Erfindung ein wesentlich verbessertes Anschneidverhalten zeigen. Damit ergibt sich durch den im Vergleich zur gesamten Schlingenlänge verkürzten isolierten Abschnitt auch bei HF-Chirurgie Generatoren mit geringem Ausgangstrom eine hinreichend hohe Stromdichte, um ein sicheres Anschneiden zu gewährleisten.

Weiterhin umfasst die Schlinge an ihrem distalen Ende wenigstens eine Gleitkufe. Die Gleitkufe ist elektrisch isoliert und damit HF-chirurgisch nicht aktiv. Sie dient zur Abstützung der Schlinge gegen das Gewebe, ohne dass hierbei das Schlingenende in das Gewebe eindringen kann. Damit kann hier die Gefahr des unerwünschten Einschneidens der Schlinge in das Gewebe wesentlich reduziert werden.

Optional ist an wenigstens einem Ende der Gleitkufe ein Schutz. Dieser kann eine Abrundung oder eine Art einer Verdickung, wie beispielsweise eine kugelförmige Ausbildung des Endes sein. Durch einen solchen Schutz kann beispielsweise eine Beschädigung eines Instrumentierungskanals eines Endoskops beim Durchschieben der Schlinge vermieden werden. Zudem kann ein Einstechen in das Gewebe beim Einsatz der Resektionsschlinge vermieden werden. In einer anderen Variante ist wenigstens ein Ende einer Gleitkufe spitz ausgebildet, so dass dieses beispielsweise zur Fixierung im Gewebe in das Gewebe eingestochen werden kann.

Vorteilhafterweise liegt das Ende wenigstens einer Gleitkufe von der Schlingenmitte oder vom Katheterende ausgehend hinter dem nicht isolierten Bereich 5 und ermöglicht somit eine mechanische Abstützung hinter diesen Bereich. Als Schlingenmitte kann hier beispielsweise der Flächenschwerpunkt der von der Schlinge umschlossenen Fläche herangezogen werden.

Bevorzugt liegt die Länge einer Gleitkufe im Bereich von 5 mm bis 40 mm, besonders bevorzugt von 10 mm bis 40 mm und ganz besonders bevorzugt von 10 mm bis 15 mm.

Bevorzugte Ausführungsformen der Gleitkufen sind mindestens 1mm oder mindestens 2mm breit und haben eine Länge von mindesten 2mm oder mindestens 3mm oder mindestens 5mm. Bevorzugt hat wenigstens eine Gleitkufe einen Umfang von wenigstens 5mm oder wenigstens 8mm. Weiterhin hat wenigstens eine Gleitkufe bevorzugt eine Gleitfläche von mindestens 2 mm² oder von mindestens 5 mm².

Die Schlingenteile umfassen selbst bevorzugt federelastischen, metallischen Rund- und/oder Flachdraht. Alternativ können Sie auch bevorzugt federelastische metallische Litze umfassen.

Vorteilhafterweise kann der nicht isolierten Schlingenabschnitt mit einer Antirutschbeschichtung oder kleinen, vorzugsweise zentripetal ausgerichteten Zähnen ausgestattet werden, was die Applikation auf bzw. um glitschige Läsionen vereinfacht.

Vorteilhafterweise können alle oben aufgeführten erfindungsgemäßen Schlingen mit einem Manipulationsgriff entsprechend WO 2013/064576 ausgestattet werden.

### Beschreibung der Zeichnungen

- Fig. 1: zeigt eine erfindungsgemäße Schlinge.
- Fig. 2: zeigt eine Schlinge mit zwei Gleitkufen.
- Fig. 3: zeigt eine Schlinge mit geraden Gleitkufen.
- Fig. 4: zeigt eine Schlinge mit einer symmetrisch angeordneten Gleitkufe.
- Fig. 5: zeigt eine Schlinge mit einer flächigen Gleitkufe.
- Fig. 6: zeigt Gefahr des Eindringens einer Schneidschlinge in eine Organwand.
- Fig. 7: zeigt die Wirkung einer Gleitkufe.

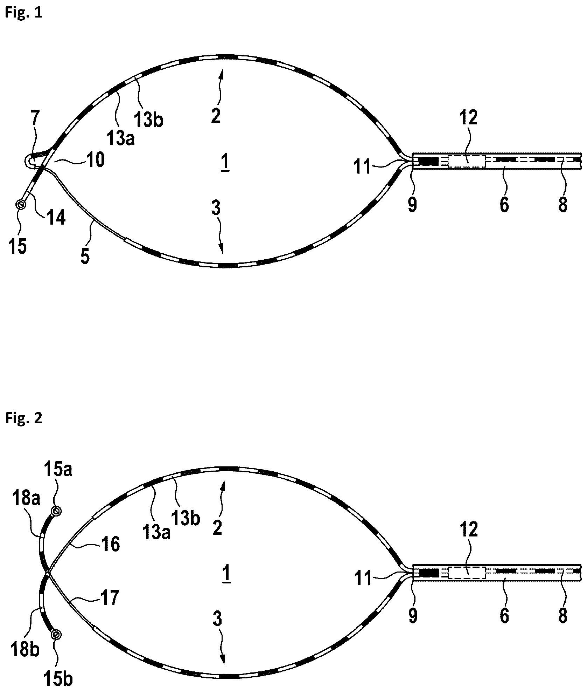

In der Figur 1 ist eine erfindungsgemäße HF-chirurgische Resektionsschlinge 1 für die flexible Endoskopie dargestellt. Sie umfasst ein erstes Schlingenteil 2 sowie ein zweites Schlingenteil 3. Diese Schlingenteile umfassen selbst bevorzugt federelastischen, metallischen Rund- und/oder Flachdraht. Alternativ können Sie auch metallische Litze umfassen. Die Schlingenteile haben jeweils ein proximales Ende 11 und ein distales Ende 10. Die proximalen Enden der Schlingenteile bilden zusammen das proximale Ende 11 der Schlinge. Sie sind mechanisch und elektrisch leitfähig, beispielsweise mit einem Verbindungselement 12, mit mindestens einem Manipulationsdraht 8 verbunden, oder als Manipulationsdraht ausgeführt. So kann beispielsweise wenigstens eines der Schlingenteile an der proximalen Seite wesentlich verlängert sein, so dass die Verlängerung als Manipulationsdraht dient. Weiterhin bilden die beiden Schlingenteile ein distales Schlingenende 10 mit einer Schlingenspitze 7. Die Schlingenspitze ist optional, wird aber meist benötigt, um eine in einen Katheter einziehbare Schlinge zu gestalten. Es können die beiden Schlingenteile auch durch einen durchgängigen Draht gebildet sein. Weiterhin sind die beiden Schlingenteile derart vorgeformt, dass sie gemeinsam eine federelastisch verformbare HF-chirurgische Schlinge 1 bilden. Durch eine solche Schlinge kann ein polypenförmiges oder flach wachsendes pathologisches Gewebeareal ohne oder mit geeigneter Vorbereitung, z.B. submuköse Unterspritzung und zirkumferentiale Umschneidung, umschlungen und von einer Organwand entfernt werden.

Weiterhin ist bevorzugt der erste Schlingenteil 2 vollständig elektrisch isoliert. Der zweite Schlingenteil 3 ist in einem proximalen Bereich elektrisch isoliert und in einem distalen Bereich 5 elektrisch nicht isoliert.

Zur besseren Bewertung des Schneidfortschritt sind bevorzugt Markierungen 13a, 13b an den Schlingenteilen und/oder dem Manipulationsdraht vorgesehen. Bevorzugt bilden diese gegeneinander einen deutlichen Farbkontrast. So kann hier die Bewegung gegenüber dem distalen Katheterende 9 gut beobachtet werden.

Eine Gleitkufe 14 geht hier von dem ersten Schlingenteil 2 aus. Sie kann ebenso von dem zweiten Schlingenteil 3 aus gehen. Die hier dargestellte Ausführungsform ist jedoch besonders vorteilhaft, da das Ende der Gleitkufe von der Schlingenmitte ausgehend hinter dem nicht isolierten Bereich 5 liegt und somit gerade eine mechanische Abstützung hinter diesen Bereich ermöglicht. Als Schlingenmitte kann hier beispielsweise der Flächenschwerpunkt der von der Schlinge umschlossenen Fläche herangezogen werden. In der hier dargestellten Ausführungsform überkreuzt die Gleitkufe 14 den nicht isolierten Bereich 5. am Ende der Gleitkufe befindet sich bevorzugt erweise ein Schutz 15. Bei weiterem zusammenziehen der Schlinge wird diese enger, so dass sich auch der erste Schlingenteil und der zweite Schlingenteil aneinander annähern. Damit verschwenkt in dieser Ausführungsform Gleitkufe 14 in eine Richtung parallel zur Längsachse des Katheters. Dies entspricht in der Darstellung entsprechend der Figur 1 einer Verschwenkung nach oben. Ist die Schlinge komplett in den Katheter eingezogen, so liegt die Gleitkufe 14 über der Katheterspitze 7.

Die Figur 2 zeigt eine weitere Ausgestaltung mit zwei Gleitkufen 18a, 18b. Diese haben an ihren Enden jeweils einen Schutz 15a, 15b. In diesem Ausführungsbeispiel hat der erste Schlingenabschnitt 2 einen ersten nicht isolierten Bereich 16 und der zweite Schlingenabschnitt 3 einen zweiten nicht isolierten Bereich 17.

Grundsätzlich können im Sinne dieser Erfindung einer oder mehrere nicht isolierte Bereiche mit den unterschiedlichen hier dargestellten Gleitkufen kombiniert werden. Besonders günstig ist es jedoch, zwei Gleitkufe zu verwenden, wenn auch zwei nicht isolierte Schlingenabschnitte, wie in der Figur 2 dargestellt, vorhanden sind.

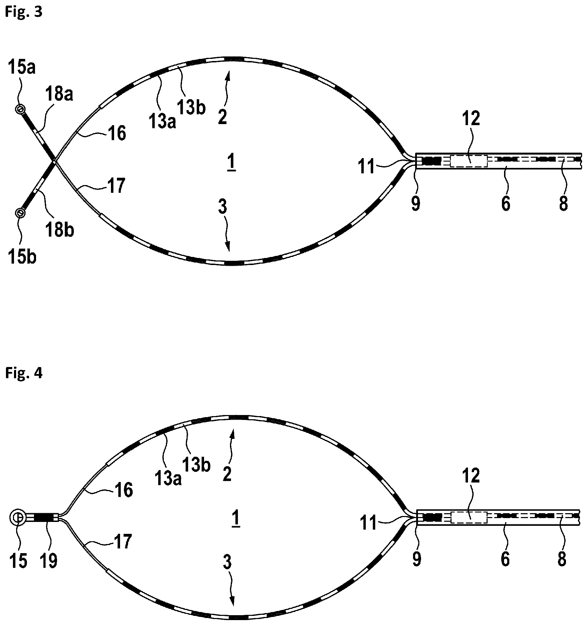

Die Figur 3 zeigt eine weitere Ausführungsform, bei der Gleitkufen gerade ausgeführt sind.

In der Figur 4 ist eine einzige symmetrisch angeordnete Gleitkufe 19 dargestellt.

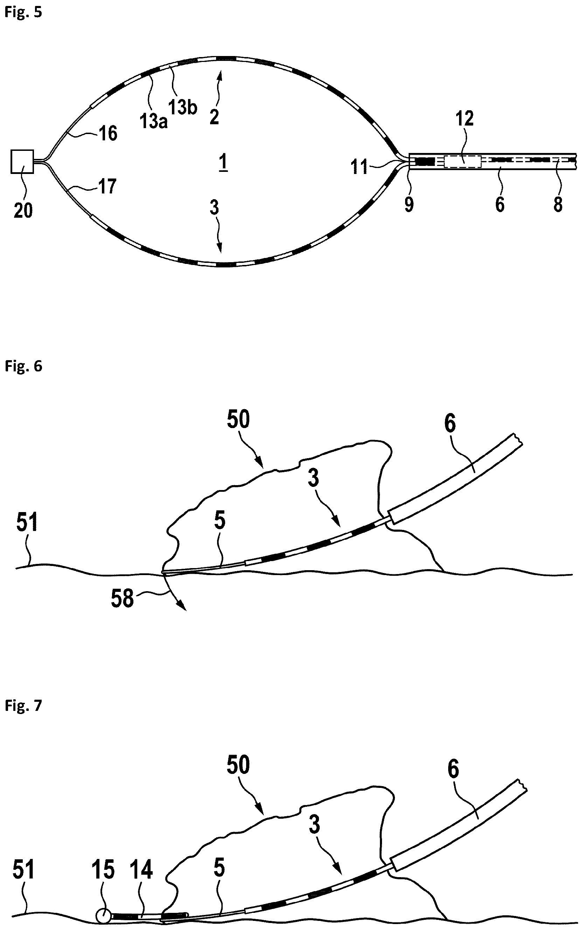

Die Figur 5 zeigt eine flächige Gleitkufe 20. Diese kann beispielsweise aufgerollt sein, um sie durch den Katheter zu führen.

In der Figur 6 ist dargestellt, wie beim Schneiden ohne Gleitkufe die Gefahr eines unerwünschten Eindringens in die Organwand entsteht. Ein pathologisches Gewebeareal 50 wird durch die Schlinge umschlossen. In der seitlichen Ansicht ist hier der zweite Schlingenabschnitt erkennbar. Meist kommt der Katheter 6 aus der Mitte des Hohlorgans, beispielsweise des Darmes und liegt somit unter einem Winkel zur Organwand 51. Damit das pathologische Areal möglichst nahe an der Organon abgetragen werden kann, muss die Schlinge gegen die Organwand gedrückt werden. Dadurch entsteht eine Kraft in der Eindringrichtung 58 auf die Organwand zu. Damit kann der nicht isolierte Bereich 5 der Schlinge nun in der Richtung 58 in die Organwand eindringen und diese beschädigen, was im schlimmsten Falle zu einer Perforation des Organs führt.

Die Figur 7 zeigt, wie durch eine Gleitkufe ein Eindringen der Schlinge in die Organwand vermieden wird. Es liegt hier Gleitkufe 14 mit ihrem Schutz 15 auf der Organwand auf und verhindert somit ein Eindringen des nicht isolierten Bereichs 5 in die Organwand hinein. Es ist offensichtlich, dass durch eine längere Gleitkufe 14 ein längerer Hebel und somit ein besserer Schutz vor dem Eindringen des nicht isolierten Bereichs 5 in die Darmwand besteht. Ebenso kann durch eine längere Gleitkufe 14 und/oder durch einen größeren Schutz 15 die Auflagefläche auf der Organwand 51 vergrößert werden. Häufig ist die Oberfläche der Organwand selbst elastisch und gibt bei Druck durch Gleitkufe zumindest geringfügig nach. Durch eine Vergrößerung der Auflagefläche kann dem entgegengewirkt werden.

### Bezugszeichenliste

- 1: Resektionsschlinge
- 2: Erster Schlingenteil
- 3: Zweiter Schlingenteil
- 5: nicht isolierter Bereich
- 6: Katheter
- 7: Katheterspitze
- 8: Manipulationsdraht
- 9: Distales Katheterende
- 10: distales Ende der Resektionsschlinge
- 11: proximales Ende der Resektionsschlinge
- 12: Verbindungselement
- 13a, 13b: Kontrastierende Markierungen
- 14: Gleitkufe
- 15: Schutz
- 16: Erster nicht isolierter Bereich
- 17: Zweiter nicht isolierter Bereich
- 18a, 18b: Gleitkufe
- 19: Gleitkufe
- 20: flächige Gleitkufe
- 50: pathologisches Gewebeareal
- 51: Organwand
- 58: Eindringrichtung

## Patentansprüche

1. Resektionsschlinge (1), umfassend einen ersten Schlingenteil (2) und einen zweiten Schlingenteil (3), die an einem distalen Ende (10) eine Schlingenspitze (7) bilden und an einem proximalen Ende (11) mit einem in einem Katheter (6) geführten Manipulationsdraht (8) verbunden sind oder in diesen übergehen,
**dadurch gekennzeichnet, dass**
die beiden Schlingenteile (2, 3) proximal elektrisch isoliert sind und wenigstens ein Schlingenteil in einem distalen Bereich (5, 16, 17) elektrisch nicht isoliert ist, und dass
die Resektionsschlinge (1) an ihrem distalen Ende (10) mindestens eine elektrisch isolierte, HF-chirurgisch nicht aktive Gleitkufe (14, 18a, 18b, 19, 20) umfasst.

2. Resektionsschlinge nach Anspruch 1,
**dadurch gekennzeichnet, dass**
der wenigstens eine elektrisch nicht isolierte Schlingenabschnitt (5) eine definierte Länge im Bereich von 10 mm bis 30 mm hat.

3. Resektionsschlinge nach Anspruch 1 oder 2,
**dadurch gekennzeichnet, dass**
wenigstens eine Gleitkufe (14, 18a, 18b, 19, 20) von der Schlingenmitte aus gesehen hinter einem elektrisch nicht isolierten distalen Bereich (5, 16, 17) liegt.

4. Resektionsschlinge nach einem der vorhergehenden Ansprüche,
**dadurch gekennzeichnet, dass**
wenigstens eines der Schlingenteile (2, 3) federelastische, metallische Litze und/oder federelastischen, metallischen Rund- und/oder Flachdraht umfasst.

5. Resektionsschlinge nach einem der vorhergehenden Ansprüche,
**dadurch gekennzeichnet, dass**
die Resektionsschlinge (1) an ihrem proximalen Ende (11) durch den Manipulationsdraht aus dem Katheter herausgeschoben und/oder in den Katheter hineingezogen werden kann.

6. Resektionsschlinge nach einem der vorhergehenden Ansprüche,
**dadurch gekennzeichnet, dass**
die Resektionsschlinge (1) an ihrem proximalen Ende (11) durch den Manipulationsdraht aus dem Katheter herausgeschoben und/oder in den Katheter hineingezogen werden kann.
